# EUROPÄISCHE PATENTANMELDUNG

(11) **EP 2 486 853 A1**
(43) Veröffentlichungstag der Anmeldung: **15.08.2012**
(21) Anmeldenummer: 12153697.3
(22) Anmeldetag: 02.02.2012
(51) Int. Cl.: A61B 6/04, A61G 13/12

(54) **Röntgenfähige Patientenlagerplatte**

(30) Priorität: 11.02.2011 DE 202011000319 U
(71) Anmelder: MAQUET GmbH & Co. KG, 76437 Rastatt (DE)
(72) Erfinder: Revenus, Rolf, 76456 Kuppenheim (DE); Dörr, Holger, 76437 Rastatt (DE)
(74) Vertreter: Schaumburg, Thoenes, Thurn, Landskron, Eckert

(57) **Zusammenfassung**

Bei einer röntgenfähige Patientenlagerplatte mit einer äußeren Schale, die einen mit einem Füllmaterial gefüllten Hohlraum umschließt, besteht die Schale aus einem wannenförmigen Oberteil (36) und einem wannenförmigen Unterteil (38), die jeweils einen die Plattenoberseite bzw. die Plattenunterseite bildenden Wannenboden (40, 42) haben, der mit dem Wannenrand (44, 46) einen Innenwinkel von mehr als 90° bildet, wobei das Oberteil (36) und das Unterteil (38) so bemessen und ineinander gesetzt sind, dass die Wannenränder (44, 46) der beiden Teile (36, 38) flach aneinander anliegen und ihre Wannenböden (40, 42) den Schalenhohlraum zwischen sich begrenzen.

## Beschreibung

Die Erfindung betrifft eine röntgenfähige Patientenlagerplatte mit einer äußeren Schale, die einen mit Füllmaterial gefüllten Hohlraum umschließt.

Heutige Operationstische in der Orthopädie haben häufig fest mit der Tragestruktur des Tisches verbundene Platten zur Lagerung eines Patienten. Dabei treten beim Durchleuchten dieser Platten immer wieder Probleme auf, die zum Teil von der Bauart der Platten selber oder von ihrer Befestigung an der Tragestruktur des Operationstisches herrühren

Der Erfindung liegt die Aufgabe zugrunde, eine röntgenfähige Patientenlagerplatte der eingangs genannten Art anzugeben, die einerseits eine stabile und bequeme Lagerung des Patienten gewährleistet und andererseits das Durchleuchten des auf der Patientenlagerplatte liegenden Patienten so wenig wie möglich behindert.

Diese Aufgabe wird erfindungsgemäß dadurch gelöst, dass die Schale aus einem wannenförmigen Oberteil und einem wannenförmigen Unterteil besteht, die jeweils einen die Plattenoberseite bzw. die Plattenunterseite bildenden Wannenboden und einen Wannenrand haben, der mit dem Wannenboden einen Innenwinkel von mehr als 90° bildet, und dass das Oberteil und das Unterteil so bemessen und ineinander gesetzt sind, dass die Wannenränder der beiden Teile flach aneinander anliegen und ihre Wannenböden den Schalenhohlraum zwischen sich begrenzen. Das Oberteil übergreift somit das Unterteil und sitzt mit der Innenfläche seines Wannenrandes auf der Außenfläche des Wannenrandes des Unterteils auf. Die resultierende Patientenlagerplatte hat somit im Querschnitt dachartig geneigte Randflächen, die zum einen für mehr Komfort bei der Lagerung des Patienten sorgen und zum anderen eine Materialanhäufung beim Durchstrahlen der Patientenlagerplatte vermeiden. Im gesamten Plattenbereich ist der Weg, den die Röntgenstrahlen innerhalb des Schalenmaterials durchlaufen, praktisch immer nur gleich der doppelten Wandstärke der Schale. Wäre die Schale in Form eine flachen quaderförmigen Kastens ausgebildet, entspräche der innerhalb des Schalenmaterials verlaufende Weg der Röntgenstrahlen im Randbereich der Schale der Plattendicke, so dass die Platte in diesem Bereich deutliche Schatten im Röntgenbild verursachen kann.

Soll die Patientenlagerplatte als Beckenlagerplatte verwendet werden, so hat sie zweckmäßigerweise zumindest annähernd die Form eines gleichschenkligen Dreiecks, so dass die breite Dreiecksbasis zur Abstützung des Beckens des Patienten zur Verfügung steht, während zur Dreiecksspitze hin die Beine des Patienten frei liegen.

Zweckmäßigerweise ist die Patientenlagerplatte so ausgebildet, dass sie leicht austauschbar ist. Hierzu hat die Patientenlagerplatte nahe ihren Rändern Aussparungen zur Aufnahme von Halteelementen, die an Auflageholmen der Tragestruktur des Operationstisches angeordnet sind. Diese Aussparungen können beispielsweise Schlitze umfassen, in welche die als Kopfbolzen ausgebildeten Halteelemente eingreifen, so dass die Patientenlagerplatte an den Auflageholmen der Tragestruktur des Operationstisches eingehängt werden kann, ohne dass an der Patientenlagerplatte selber metallische Befestigungselemente angebracht werden müssten.

Vorzugsweise bestehen die Schalenteile, d.h. das Oberteil und das Unterteil aus einem Faserverbundstoff, beispielsweise kohlefaserverstärktem Kunststoff, während der Hohlraum der Schale mit einem Schaumstoff, beispielsweise einem Hartschaum ausgefüllt ist. Auf diese Weise erhält man eine formstabile, sehr belastbare Lagerplatte, die röntgenfähig ist und leicht ausgetauscht werden kann. Die folgende Beschreibung erläutert in Verbindung mit den beigefügten Zeichnungen die Erfindung anhand eines Ausführungsbeispiels. Es zeigen:
- Fig.1: eine teilweise schematische perspektivische Darstellung eines Teils einer Patientenlagerfläche für einen Orthopädie-Operationstisch,
- Fig.2: eine vergrößerte Darstellung des in der Fig. 1 mit A bezeichneten Konstruktionsdetails und
- Fig.3: einen Schnitt durch die Beckenlagerplatte entlang der Linie III-III in Fig.1.

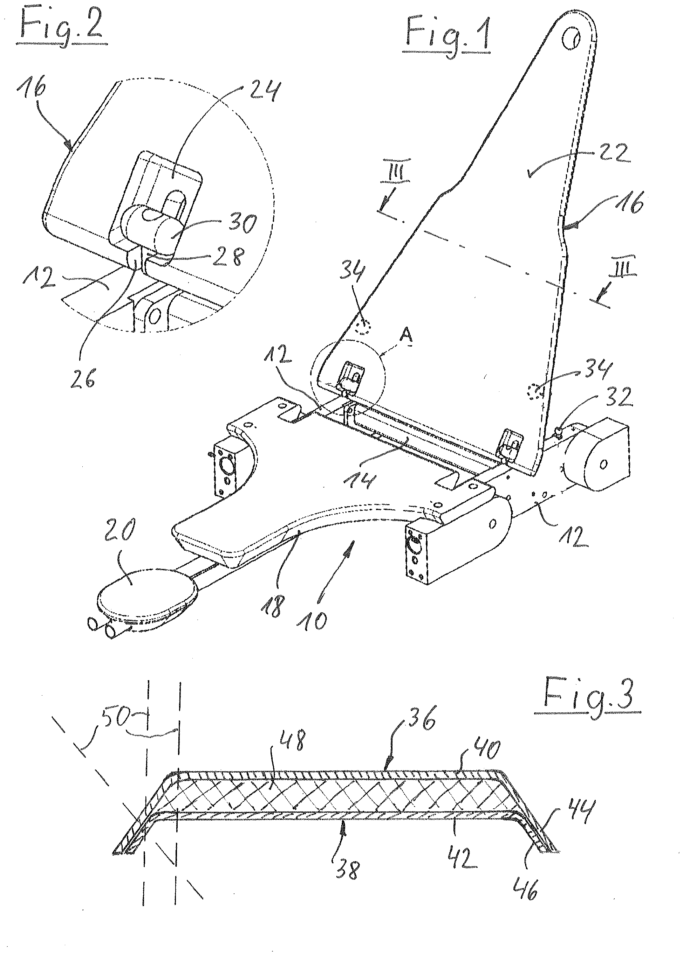

Die in der Fig.1 dargestellte allgemein mit 10 bezeichnete Patientenlagerfläche umfasst zwei Tragholme 12, die durch Querstreben 14 miteinander verbunden sind und an denen eine Beckenlagerplatte 16, eine Rückenlagerplatte 18 und eine Kopfplatte 20 angeordnet sind. Die Beckenlagerplatte 16 hat annähernd die Form eines gleichseitigen Dreiecks und ist nahe der Dreiecksbasis mit den Tragholmen 12 lösbar verbunden. Hierzu sind in der Beckenlagerplatte 16 nahe der Dreiecksbasis zwei zur Plattenoberseite 22 hin offene muldenförmige Aussparungen 24 vorgesehen, die jeweils einen zur Plattenbasis hin offenen Aufnahmeschlitz 26 haben. In diesen Aufnahmeschlitz 26 kann jeweils der Schaft 28 eines mit einem der Tragholme 12 verbundenen Kopfbolzens eingreifen, dessen Kopf 30 dann in der Mulde 24 der Lagerplatte 22 liegt (Fig.2). In der in der Fig.1 dargestellten hochgeklappten Stellung kann die Beckenlagerplatte 16 mit den Aufnahmeschlitzen 26 auf die Schäfte 28 der beiden Kopfbolzen aufgeschoben und anschließend nach unten geklappt werden, so dass sie in einer Ebene mit der Rückenplatte 18 liegt.

Dabei greifen zwei weitere Bolzen 32 an den Tragholmen 12 in jeweils eine an der Unterseite der Beckenlagerplatte 16 vorgesehen Öffnung 34 ein. Im heruntergeklappten Zustand kann die Beckenlagerplatte somit nicht verschoben werden. Sie wird in dieser Lage durch das Eigengewicht des auf ihr liegenden Patienten gehalten. Die vorstehende Beschreibung zeigt, dass die Platte auf diese Weise rasch ausgetauscht werden kann, ohne dass irgendwelche Verriegelungsmittel gelöst oder festgezogen werden müssen.

Der Aufbau der Patientenlagerplatte 16 ist in Fig.3 zu erkennen. Die Platte besteht aus einem formstabilen wannenförmigen Oberteil 36 und einem wannenförmigen formstabilen Unterteil 38, die zusammen die äußere Schale der Lagerplatte 16 bilden. Jede dieser Wannen hat einen Wannenboden 40 bzw. 42 und einen gegenüber diesem nach außen geneigten Wannenrand 44 bzw. 46. Die beiden wannenförmigen Teile 36 und 38 sind so bemessen und ineinander gesetzt, dass ihre Wannenränder 44 und 46 flach aneinander liegen und die Wannenböden 40 und 42 einen Hohlraum zwischen sich einschließen, der mit einem Schaumstoff 48 gefüllt ist.

Wie man in Fig.3 erkennt, führt dieser Aufbau der Patientenlagerplatte 16 dazu, dass beim Durchleuchten die Durchleuchtungsstrahlen 50 einen Weg in dem relativ dichten Schalenmaterial zurücklegen, der im Wesentlichen maximal der doppelten Wandstärke der Schale entspricht. Damit erhält man eine gleichbleibende Dämpfung der Röntgenstrahlen über die gesamte Plattenfläche hin.

## Patentansprüche

1. Röntgenfähige Patientenlagerplatte mit einer äußeren Schale, die einen mit einem Füllmaterial gefüllten Hohlraum umschließt, **dadurch gekennzeichnet, dass** die Schale aus einem wannenförmigen Oberteil (36) und einem wannenförmigen Unterteil (38) besteht, die jeweils einen die Plattenoberseite bzw. die Plattenunterseite bildenden Wannenboden (40, 42) haben, der mit dem Wannenrand (44, 46) einen Innenwinkel von mehr als 90° bildet, und dass das Oberteil (36) und das Unterteil (38) so bemessen und ineinander gesetzt sind, dass die Wannenränder (44, 46) der beiden Teile (36, 38) flach aneinander anliegen und ihre Wannenböden (40, 42) den Schalenhohlraum zwischen sich begrenzen.

2. Patientenlagerplatte nach Anspruch 1, **dadurch gekennzeichnet, dass** sei als Beckenlagerplatte (16) ausgebildet ist und zumindest annähernd die Form eines gleichschenkligen Dreiecks hat.

3. Patientenlagerplatte nach Anspruch 1 oder 2, **dadurch gekennzeichnet, dass** die Patientenlagerplatte nahe einem ihrer Ränder Aussparungen (24, 26) zur Aufnahme von Halterungselementen (28, 30) hat, die an Tragholmen (12) einer Patientenlagerfläche angeordnet sind.

4. Patientenlagerplatte nach einem der Ansprüche 1 bis 3, **dadurch gekennzeichnet, dass** das Oberteil (36) und das Unterteil (38) der Schale aus einem Faserverbundstoff bestehen.

5. Patientenlagerplatte nach einem der Ansprüche 1 bis 4, **dadurch gekennzeichnet, dass** das Füllmaterial aus Schaumstoff, insbesondere einem Kunststoffhartschaum besteht.
